# EUROPÄISCHE PATENTSCHRIFT

(11) **EP 1 238 273 B1**
(45) Veröffentlichungstag und Bekanntmachung des Hinweises auf die Patenterteilung: **06.08.2008**
(21) Anmeldenummer: 01949349.3
(22) Anmeldetag: 18.05.2001
(51) Int. Cl.: G01N 33/36, D02G 1/02, B65H 63/00

(54) **VERFAHREN ZUR STEUERUNG EINER TEXTURIERMASCHINE SOWIE EINE TEXTURIERMASCHINE**
METHOD FOR CONTROLLING A TEXTURING MACHINE, AND TEXTURING MACHINE
PROCEDE POUR COMMANDER UNE MACHINE A TEXTURER, ET MACHINE A TEXTURER

(30) Priorität: 30.05.2000 DE 10026942
(43) Veröffentlichungstag der Anmeldung: 11.09.2002
(73) Patentinhaber: Oerlikon Textile GmbH & Co. KG, 42897 Remscheid (DE)
(72) Erfinder: WORTMANN, Thomas, 42105 Wuppertal (DE); PYRA, Michael, 41379 Brüggen (DE); LIEBER, Reinhard, 45549 Sprockhövel (DE); KLUG, Michael, 44892 Bochum (DE); GEISSLER, Stefan, 42553 Velbert (DE)
(74) Vertreter: Kahlhöfer, Hermann
(86) Internationale Anmeldenummer: PCT/EP2001/005687
(87) Internationale Veröffentlichungsnummer: WO 2001/092615

(56) Entgegenhaltungen:
- EP-A- 0 389 849
- WO-A-98/33963
- DE-A- 3 005 746
- DE-A- 3 324 243
- DE-A- 19 710 229
- US-A- 4 720 702
- US-A- 4 720 806
- US-A- 5 515 266

## Beschreibung

Die Erfindung betrifft ein Verfahren zur Steuerung einer Texturiermaschine gemäß dem Oberbegriff des Anspruchs 1 sowie eine Texturiermaschine nach dem Oberbegriff des Anspruchs 10.

Ein gattungsgemäßes Verfahren sowie eine Texturiermaschine sind aus der WO 98/033963 bekannt.

Derartige Texturiermaschinen weisen eine Vielzahl von Bearbeitungsstellen - üblicherweise bis zu 216 oder mehr Bearbeitungsstellen - auf, die in einer Maschinenlängsseite nebeneinander angeordnet sind. Dabei weist jede der Bearbeitungsstellen mehrere Prozeßaggregate auf, die jeweils einen Prozeßschritt ausführen, um den Faden zu fördern, zu texturieren, zu verstrecken und zu einer Spule aufzuwickeln. Die Prozeßaggregate und damit die Prozeßschritte in den einzelnen Bearbeitungsstellen werden über eine zentrale Maschinensteuerung überwacht und gesteuert. Zur Bedienung weist die Steuereinrichtung in der Regel eine bedienbare Visualisiereinheit auf, durch welche Steuerungsdaten und Überwachungsdaten visualisierbar und änderbar sind. Es ist jedoch auch üblich jeweils zur Steuerung und zur Prozeßüberwachung zwei getrennte Visualisiereinheiten zu verwenden. Um in die Prozeßschritte eingreifen zu können, sind mehrere Prozeßsteuergeräte vorgesehen, die alle mit der zentralen Maschinensteuereinheit gekoppelt sind. Die Anbindung der Prozeßsteuergeräte an die Prozeßaggregate kann dabei nach zwei Möglichkeiten erfolgen. Wie aus der WO 98/033963 bekannt ist, lassen sich die Prozeßaggregate innerhalb einer Bearbeitungsstelle durch Einzelantriebe mit jeweils zugeordneten Steuergeräten betreiben, so daß jeder Bearbeitungsstelle mehrere Prozeßsteuergeräte zugeordnet sind. Dabei sind alle Prozeßsteuergeräte aller Bearbeitungsstellen mit einer zentralen Maschinensteuereinheit verbunden.

Bei einer zweiten Variante, die beispielsweise aus der DE 33 24 243 bekannt ist, werden die Prozeßaggregate gleicher Funktion aller Bearbeitungsstellen durch einen zentralen Antrieb angetrieben, so daß alle Prozeßaggregate gleicher Funktion durch ein Prozeßsteuergerät steuerbar sind. Bei dieser Variante vermindert sich der Steuerungsaufwand gegenüber der vorhergehenden Variante mit Einzelantrieben erheblich, jedoch mit dem Nachteil, daß alle Bearbeitungsstellen der Texturiermaschine synchron betrieben werden müssen.

Demgemäß ist es Aufgabe der Erfindung, ein Verfahren zur Steuerung einer Texturiermaschine zum Falschdralltexturieren einer Vielzahl von synthetischen Fäden in einer entsprechenden Vielzahl von Bearbeitungsstellen sowie eine derartige Texturiermaschine zu schaffen, bei welchem/welcher eine hohe Flexibilität in der Bearbeitung der Fäden bei gleichzeitig geringem Steuerungsaufwand erreicht wird.

Diese Aufgabe wird erfindungsgemäß durch ein Verfahren mit den Merkmalen des Anspruchs 1 sowie durch eine Texturiermaschine mit den Merkmalen des Anspruchs 10 gelöst.

Die Erfindung basiert darauf, daß die Texturiermaschine in mehrere Sektionen aufgeteilt ist, wobei jede der Sektionen unabhängig voneinander gesteuert wird.
So ist eine Texturiermaschine mit beispielsweis insgesamt 216 Bearbeitungsstellen beispielsweise in insgesamt 18 Sektionen aufgeteilt. In jeder der 18 Sektionen wären somit 12 Bearbeitungsstellen enthalten. Die Bearbeitungsstellen einer Sektion werden unabhängig von dem Bearbeitungsstellen der benachbarten Sektionen überwacht und gesteuert. Der besondere Vorteil der Erfindung liegt darin, daß mit einer Texturiermaschine Garne mit unterschiedlichen Einstellungen in den Sektionen hergestellt werden können. Desweiteren läßt sich jeder der Sektionen unabhängig von den benachbarten Sektionen zur Wartung und Fehlerbehebung abschalten. Der Produktionsausfall wird somit minimiert. Ein weiterer Vorteil ist darin zu sehen, daß alle Sektionen gemeinsam aus einer Einspeisung der Versorgungsspannung der Texturiermaschine versorgt werden. Insoweit ist es besonders vorteilhaft, die Sektionen gemeinsam an einem Hauptschalter oder einem Not-Aus-Schalter anzuschließen. -

Um innerhalb der Sektion auf allen Bearbeitungsstellen im wesentlichen gleiche Prozeßschritte ausführen zu können, sind gemäß einer vorteilhaften Weiterbildung des erfindungsgemäßen Verfahrens nach Anspruch 2 alle Prozeßschritte der Sektion durch eine der Sektion zugeteilten Feldsteuereinheit überwacht und gesteuert. Hierzu sind alle für den Prozeß relevanten Parameter in der Feldsteuereinheit hinterlegt. Ebenso werden die Betriebsdaten aus den Bearbeitungsstellen fortlaufend der Feldsteuereinheit zugeführt. Somit wird durch die Feldsteuereinheit eine individuelle Überwachung und Steuerung der Sektion möglich.

Zur Ansteuerung der Prozeßschritte wird gemäß der vorteilhaften Weiterbildung nach Anspruch 3 jeder Prozeßschritt einer der Bearbeitungsstellen unabhängig von den benachbarten Prozeßschritten der Bearbeitungsstelle einzeln gesteuert. Insbesondere die als Lieferwerke eingesetzten Prozeßaggregate können bei dieser Verfahrensvariante auf einfache Weise mit unterschiedlichen Fördergeschwindigkeiten betrieben werden. So ist es von Vorteil, wenn beim Anlegen eines Fadens in einer Bearbeitungsstelle alle Lieferwerke eine gleich hohe Fördergeschwindigkeit aufweisen. Erst nachdem der Faden angelegt ist, erfolgt die Differenzbildung in den Fördergeschwindigkeiten, die zu einem Verstrecken des Fadens führt.

Eine besonders hohe Flexibilität in der Bearbeitung der einzelnen Fäden innerhalb der Sektion wird durch die Verfahrensvariante gemäß Anspruch 4 erreicht. Hierbei werden die Prozeßschritte einer Bearbeitungsstelle unabhängig von den Prozeßschritten der benachbarten Bearbeitungsstellen einzeln gesteuert.

Es besteht jedoch auch die Möglichkeit, daß die Prozeßschritte - insbesondere die mit gleicher Funktion - benachbarter Bearbeitungsstellen unabhängig von den jeweils benachbarten Prozeßschritten der Bearbeitungsstelle als eine Gruppe gesteuert werden. So lassen sich beispielsweise zum Abziehen der Fäden von einer Vorlagespule die ersten Lieferwerke der Bearbeitungsstellen einer Sektion gemeinsam ansteuern.

Die Weiterbildung des erfindungsgemäßen Verfahrens nach Anspruch 6 ist insbesondere geeignet, um die verschiedenen Arten von Prozeßschritten innerhalb einer Bearbeitungsstelle mit möglichst geringem Steuerungsaufwand zu betreiben. Als Prozeßschritte sind hierbei beispielsweise das Fördern, das Erwärmen, das Verstrecken, das Verdrallen und das Aufwickeln vorgesehen. So könnten die Prozeßschritte, die zum Fördern des Fadens ausgeführt werden, vorteilhaft als eine Gruppe gesteuert werden. Dagegen werden die Prozeßschritte zum Verdrallen eines Fadens vorzugsweise einzeln gesteuert.

Für den Fall, daß Prozeßschritte mehrerer Bearbeitungsstellen einer Sektion zu einer Gruppe zusammengeführt sind, ist die Weiterbildung des Verfahrens gemäß Anspruch 7 besonders vorteilhaft um unabhängig von der Steuerung ein Abschalten der Prozeßschritte zu ermöglichen. Ein derartiges Abschalten wird beispielsweise bei einem Fadenbruch innerhalb einer Bearbeitungsstelle oder durch Überschreiten eines fortlaufend überwachten Parameters innerhalb der Bearbeitungsstelle notwendig.

Das erfindungsgemäße Verfahren basiert auf Texturiermaschine mit sehr vielen Bearbeitungsstellen, so daß innerhalb einer Sektion mindestens sechs Fäden vorzugsweise mindesten zwölf Fäden gleichzeitig paralle nebeneinander bearbeitet werden.

Die erfindungsgemäße Texturiermaschine mit den Merkmalen nach Anspruch 10 stellt die vorrichtungsgemäße Lösung der zugrunde liegenden Aufgabe dar. Hierzu ist die Vielzahl der Bearbeitungsstellen in mehrere Sektionen mit jeweils mehreren Bearbeitungsstellen aufgeteilt. Jeder Sektion ist eine von mehreren Feld-Feldsteuereinheiten zur Steuerung und Überwachung der Bearbeitungsstellen der Sektion zugeordnet. Die Prozeßsteuergeräte zur Steuerung der Prozeßaggregate der Bearbeitungsstellen einer Sektion sind dabei mit der zugeordneten Feldsteuereinheit verbunden. Jede Sektion mit zugeordneter Feldsteuereinheit bildet somit eine Teilmaschine, die individuell zu betreiben ist. Die Prozeßparameter werden hierbei durch die Feldsteuereinheit vorgegeben.

Zur Bedienung der Texturiermaschine sind die Feldsteuereinheiten unabhängig voneinander mit einem Mikroprozessor verbunden, so daß eine Bedienperson die Daten einer oder mehrerer Feldsteuereinheit zur Anzeige bringen oder eine Dateneingabe oder eine Datenänderung an einer bedienbaren Visualisiereinheit vornehmen kann.

Zur Erhöhung der Effizienz einer Texturiermaschine wird weiter vorgeschlagen, daß der Mikroprozessor über ein Ferndatennetz mit einer Diagnosestation koppelbar ist. Dadurch besteht die Möglichkeit eines schnellen Eingriffs einer zentralen Station bei Auftreten eines Fehlers.

Zur Datenübertragung zwischen der Feldsteuereinheit und den Prozeßsteuergeräten sowie zwischen den Prozeßsteuergeräten und der Feldsteuereinheit ist vorteilhaft ein serielles Bussystem vorgesehen. Damit sind schnelle Einstellungsänderungen ausführbar.

Aufgrund unterschiedlicher Anforderungen einzelner Prozeßschritte sind die Menge und die Häufigkeit des Datenaustauschs zwischen den Prozeßaggregaten und der Feldsteuereinheit unterschiedlich. Hierzu kann das Bussystem vorteilhaft aus mehreren Datennetzwerken bestehen, die unterschiedliche Datenübertragungsraten aufweisen.

Um individuelle Einstellungsveränderungen in den Prozeßschritten der Bearbeitungsstellen vornehmen zu können, ist gemäß einer vorteilhaften Weiterbildung der erfindungsgemäßen Texturiermaschine jedem der Prozeßaggregate der Bearbeitungsstellen einer Sektion eine einzelnes der Prozeßsteuergeräte zugeordnet.

Die Prozeßschritte innerhalb der Bearbeitungsstellen, die keine größeren Änderungen erhalten, können vorteilhaft gemäß der Weiterbildung nach Anspruch 16 ausgeführt sein. Hierbei sind die Prozeßaggregate der Barbeitungsstellen einer Sektion in Gruppen gleicher Funktion aufgeteilt und jeder Gruppe von Prozeßaggregaten ist ein einzelnes Prozeßsteuergerät zugeordnet.

Um trotz der gruppenmäßigen Zusammenfassung einzelner Prozeßschritte benachbarter Bearbeitungsstellen die Flexibilität in der Bearbeitung der Fäden beizubehalten, ist gemäß einer Weiterbildung der erfindungsgemäßen Texturiermaschine nach Anspruch 18 zumindest einem Teil der Prozeßaggregate ein Schalter zugeordnet, durch welchen die Prozeßaggregate schaltbar mit einer der Prozeßsteuergeräte verbunden sind, wobei der Schalter unabhängig von dem Prozeßsteuergerät ansteuerbar ist.

Eine derartige Texturiermaschine ist jedoch auch für sich geeignet, um die der Erfindung zugrunde liegende Aufgabe zu lösen. Durch die gruppenweise angesteuerten Prozeßaggregate kann durch die schaltbare Verbindung zwischen den Prozeßaggregaten und den Prozeßsteuergeräten vorteilhaft jede der Barbeitungsstellen individuell gesteuert werden. Tritt beispielsweise in einer Gruppe von gemeinsam gesteuerten Lieferwerken in einem der Lieferwerke ein Fadenwickel auf, so läßt sich das betreffende Lieferwerk von dem Prozeßsteuergerät abschalten, ohne daß dabei die benachbarten Lieferwerke in ihrer Ansteuerung beeinflußt werden.

Die zwischen den Prozeßaggregaten und den Prozeßsteuergeräten vorgesehenen Schalter lassen sich vorteilhaft durch die übergeordnete Feldsteuereinheit ansteuern.

Es ist jedoch auch möglich, daß den Prozeßaggregaten eine Überwachungseinheit zugeordnet ist, durch welche die Schalter im Bedarfsfall aktiviert werden.

Um ein Abschalten eines Prozeßaggregates im Bedarfsfall schnell und ohne größere Verzögerung ausführen zu können, ist gemäß einer vorteilhaften Weiterbildung eine Sensorik zur Überwachung zumindest eines Parameters des Prozeßaggregates vorgesehen. Die Sensorik ist mit der an dem Prozeßaggregat zugeordneten Überwachungseinheit oder mit der dem Prozeßaggregat zugeordneten Feldsteuereinheit verbunden. Als Parameter können hierbei die Temperatur eines Motors, die durch einen Temperatursensor unmittelbar am Motor erfaßt wird, oder eine Solldrehzahl eines Antriebes, die unmittelbar durch einen Drehzahlsensor an einem Antrieb erfaßt wird, beispielsweise gewählt werden. Es ist jedoch auch möglich, das Abschalten unmittelbar durch ein Signal der Feldsteuereinheit oder der Überwachungseinheit auszulösen, die aufgrund eines Signals eines Fadenwächters erfolgt.

Die Steuerungsfunktionen und die Überwachungsfunktionen werden vorteilhaft von einer bedienbaren Visualisiereinheit durch eine Bedienperson ausgeführt, wobei die Visualisiereinheit mit dem Mikroprozessor verbunden ist. Es ist jedoch auch möglich, die Steuerungsfunktionen und die Überwachungsfunktionen von zwei getrennten Visualisiereinheiten auszuführen.

Gemäß einer bevorzugten Weiterbildung der Erfindung enthalten die Sektionen jeweils mindesten sechs Bearbeitungsstellen vorzugsweise zwölf Bearbeitungsstellen.

Unter Hinweis auf die beigefügten Zeichnungen sind das erfindungsgemäße Verfahren sowie einige Ausführungsbeispiele der erfindungsgemäßen Texturiermaschine nachfolgend näher beschrieben.

Es stellen dar:
- Fig. 1: eine schematische Seitenansicht eines ersten Ausführungsbeispiels einer erfindungsgemäßen Texturiermaschine;
- Fig. 2: schematisch eine Draufsicht auf das Ausführungsbeispiel nach Fig. 1;
- Fig. 3: schematisch die Steuerung einer Sektion der Texturiermaschine aus Fig. 1 und 2;
- Fig. 4: schematisch ein weiteres Ausführungsbeispiel einer Ansteuerung einer Sektion der erfindungsgemäßen Texturiermaschine;
- Fig. 5 und 6: weitere Ausführungsbeispiele der Ansteuerung einer Sektion der erfindungsgemäßen Texturiermaschine.

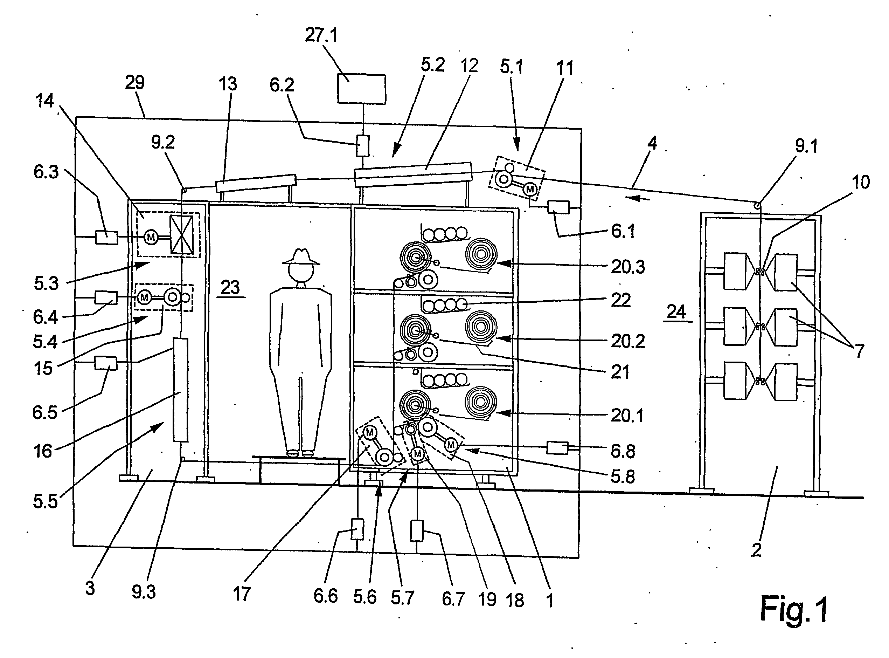

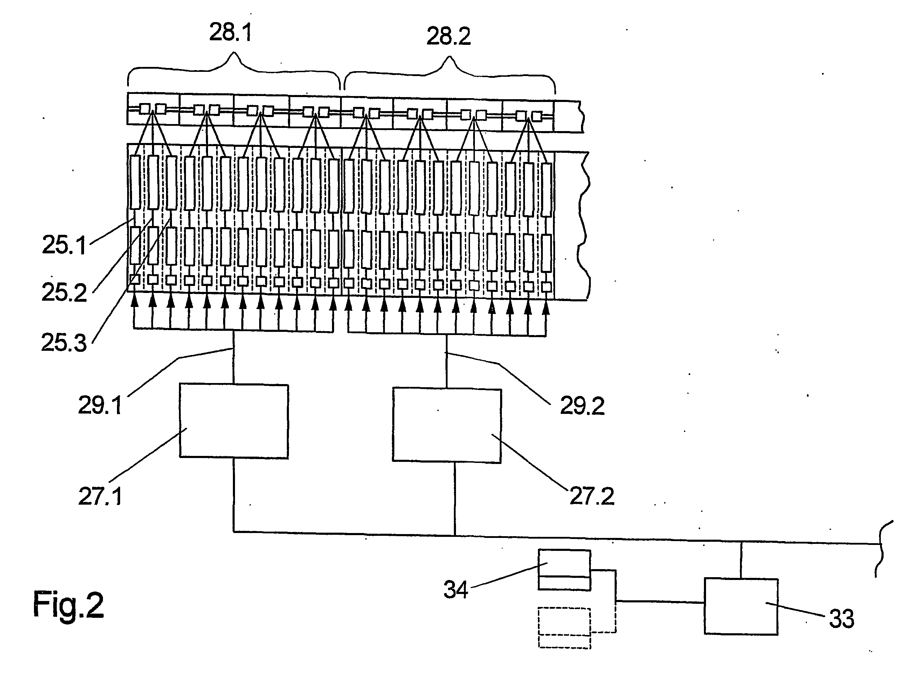

In Fig. 1 ist schematisch eine Seitenansicht einer erfindungsgemäßen Texturiermaschine und in Fig. 2 schematisch eine Draufsicht auf die erfindungsgemäße Texturiermaschine dargestellt. Insoweit kein ausdrücklicher Bezug zu einer der Figuren gemacht ist, gilt die nachfolgende Beschreibung für beide Figuren.

Die Texturiermaschine besteht aus einem Gattergestell 2, einem Prozeßgestell 3 und einem Wickelgestell 1. Zwischen dem Prozeßgestell 3 und dem Wickelgestell 1 ist ein Bediengang 23 gebildet. Auf der zum Bediengang 23 gegenüberliegenden Seite des Wickelgestells 1 ist das Gattergestell 2 mit Abstand zum Wickelgestell 1 angeordnet. Zwischen dem Wickelgestell 1 und dem Gattergestell 2 wird somit ein Doffgang 24 gebildet.

Die Texturiermaschine weist in Längsrichtung eine Vielzahl von Bearbeitungsstellen 25 auf. Üblicherweise sind in einer Texturiermaschine über 200 Bearbeitungsstellen, von denen in Fig. 2 beispielhaft die ersten drei Bearbeitungsstellen mit den Bezugszeichen 25.1, 25.2 und 25.3 gekennzeichnet sind. In jeder der Bearbeitungsstellen 25 wird jeweils ein Faden bearbeitet. Die Aufwickeleinrichtungen nehmen eine Breite von drei Bearbeitungsstellen ein. Daher sind jeweils drei Aufwickeleinrichtungen 20.1, 20.2 und 20.3 - hierauf wird später eingegangen - in einer Säule übereinander im Wickelgestell 1 angeordnet.

Jede Bearbeitungsstelle 25 weist eine Vorlagespule 7 auf, auf der ein thermoplastischer Faden 4 aufgewickelt ist. Der Faden 4 wird durch mehrere in Fadenlaufrichtung hintereinander angeordnete Prozeßaggregate 5.1 bis 5.8 in mehreren Prozeßschritten behandelt und zu einer Spule aufgewickelt. Jedem der Prozeßaggregate 5.1 bis 5.8 ist jeweils ein Prozeßsteuergerät 6.1 bis 6.8 zugeordnet. Die Prozeßsteuergeräte 6.1 bis 6.8 sind über ein Bussystem 29 mit einer Feldsteuereinheit 27.1 gekoppelt.

Im einzelnen werden die Prozeßaggregate bei der in Fig. 1 und 2 gezeigten Texturiermaschine durch ein erstes Lieferwerk 11, eine Heizeinrichtung 12, ein Falschdralltexturieraggregat 14, ein zweites Lieferwerk 15, einen Set-Heizer 16, ein drittes Lieferwerk 17, eine Changiereinrichtung 19 und einen Spulenantrieb 18 gebildet. Hierbei wird der Faden 4 in einer Bearbeitungsstelle 25 über einen Kopffadenführer 10 und eine Umlenkrolle 9.1 unter einer gewissen Spannung durch das erste Lieferwerk 11 abgezogen. Das erste Lieferwerk 11 weist einen Motor und eine mit dem Motor verbundene Lieferwelle auf. Die Lieferwelle kann beispielsweise als eine Förderrolle mit einer am Umfang zick-zack-förmigen Fadenlaufspur ausgebildet sein, wie sie aus der WO 98/033963 bekannt ist. Es ist jedoch auch möglich, die Lieferwelle als Galette auszuführen, die mehrfach von dem Faden umschlungen ist. Unabhängig von der Ausführung der Lieferwelle ist jeweils zum Antrieb ein Elektromotor vorgesehen. Das Prozeßsteuergerät 6.1 zur Steuerung des Motors ist somit als Umrichter ausgebildet

In Fadenlaufrichtung hinter dem ersten Lieferwerk 11 befindet sich ein erster langgestreckter Heizer 12, durch welchen der Faden 4 läuft, wobei der Faden auf eine bestimmte Temperatur erwärmt wird. Der Heizer könnte als Hochtemperaturheizer ausgeführt sein, bei dem die Heizeroberflächentemperatur über 300°C liegt. Ein derartiger Heizer ist beispielsweise aus der EP 0 412 429 (Bag. 1720) bekannt. Insoweit wird auf diese Druckschrift Bezug genommen. Zur Temperaturkontrolle ist in dem Heizer 12 das Prozeßsteuergerät 6.2 zugeordnet, das in diesem Fall als eine Heizsteuerung ausgebildet ist.

Hinter dem Heizer 12 befindet sich eine Kühlschiene 13. Hinter der Kühlschiene 13 ist ein Falschdrallaggregat 14 in dem Prozeßgestell 3 angeordnet. Dieses Falschdrallaggregat 14 kann als Friktionsscheibenaggregat mit einem Einzelmotorantrieb nach EP 0 744 480 (Bag. 2322) ausgebildet sein. Dem Motor des Falschdrallaggregates 14 ist das Prozeßsteuergerät 6.3 -beispielsweise ein Umrichter - zugeordnet.

Im Anschluß an das Falschdrallaggregat 14 dient ein zweites weiteres Lieferwerk 15 dazu, den Faden 4 sowohl über den Heizer 12 als auch die Kühlschiene 13 zu ziehen. Das zweite Lieferwerk 15 wird über das Prozeßsteuergerät 6.4 gesteuert. Das Lieferwerk 15 könnte dabei den gleichen Aufbau wie das zuvor beschriebene erste Lieferwerk 11 aufweisen.

In Fadenlaufrichtung hinter dem zweiten Lieferwerk 15 befindet sich ein Set-Heizer 16, dem ebenfalls ein Prozeßsteuergerät 6.5 zugeordnet ist. Hinter dem Set-Heizer 16 befindet sich ein weiteres drittes Lieferwerk 17, das den Faden 4 von der Heizeinrichtung 16 zu der Aufwickeleinrichtung 20.1 führt. Dem Lieferwerk 17 ist das Prozeßsteuergerät 6.6 zugeordnet.. Die Aufwickeleinrichtung 20.1 ist in dem Wickelgestell 1 angeordnet. In der Aufwickeleinrichtung 20.1 wird der Faden auf eine Spule aufgewickelt. Die Spule wird durch einen Spulenantrieb 18 mit im wesentlichen konstanter Umfangsgeschwindigkeit angetrieben. Der Spulenantrieb weist hierzu eine Treibwalze auf, die unmittelbar am Umfang der Spule anliegt. Die Treibwalze wird durch einen Motor angetrieben, der mit dem Prozeßsteuergerät 6.8 gekoppelt ist. Bevor der Faden auf die Spule aufläuft, wird der Faden 4 durch eine Changiervorrichtung 19 hin- und hergeführt, so daß eine Kreuzwicklung auf der Spule entsteht. Die Changiereinrichtung weist ebenfalls einen Antrieb auf, der über das Prozeßsteuergerät 6.7 angesteuert wird.

Die Aufwickeleinrichtung 20.1 weist einen Spulenspeicher 21 auf, der zur Aufnahme der vollen Spulen dient, wenn auf der Aufspuleinrichtung eine volle Spule erzeugt worden ist. Zur Abnahme der vollen Spule wird ein Spindelträger verschwenkt und die volle Spule in den Spulenspeicher 21 abgelegt. In dem Spulenspeicher 21 wartet die volle Spule bis zum Abtransport. Deswegen ist der Spulenspeicher 22 auf der Seite des Wickelgestells 1 angeordnet, welche zum Doffgang 24 benachbart und vom Bediengang 23 abgekehrt ist. Ferner ist jede Aufspuleinrichtung 20 mit einer Hülsenzufuhreinrichtung 22 ausgestattet, die im einzelnen nicht mehr beschrieben ist.

Die benachbarten Bearbeitungsstellen 25.2, 25.3 usw. weisen ebenfalls die zuvor beschriebenen Prozeßaggregate 5.1 bis 5.8 und die zugeordneten Prozeßsteuergeräte 6.1 bis 6.8 auf. Wie in Fig. 2 gezeigt, sind die Bearbeitungsstellen in einer Längsrichtung hintereinander angeordnet. Die Vielzahl der Bearbeitungsstellen der Texturiermaschine sind in mehrere Sektionen 28.1, 28.2 usw. aufgeteilt. In Fig. 2 sind zwei vollständige Sektionen 28.1 und 28.2 gezeigt. Die Sektionen 28.1 und 28.2 enthalten mehrere Bearbeitungsstellen. In diesem Beispiel bilden zwölf nebeneinander liegende Bearbeitungsstellen 25 eine Sektion 28. Jeder Sektion 28 ist eine Feldsteuereinheit 27 zugeordnet, so daß die Sektion 28.1 durch die Feldsteuereinheit 27.1 und die Sektion 28.2 durch die Feldsteuereinheit 27.2 gesteuert wird. Hierbei sind die Prozeßsteuergeräte 6 der Bearbeitungsstellen 25 einer Sektion 28 mit der jeweils zugeordneten Feldsteuereinheit 27 durch ein Bussystem 29 verbunden. In dem Ausführungsbeispiel ist die Feldsteuereinheit 27.1 über das Bussystem 29.1 mit den Prozeßsteuergeräten 6.1 bis 6.8 der Sektion 28.1 verbunden. Die Feldsteuereinheit 27.2 ist über das Bussystem 29.2 mit den Prozeßsteuergeräten 6 der Sektion 28.2 verbunden. In dieser Weise sind alle Bearbeitungsstellen der Texturiermaschine aufgeteilt.

Die Feldsteuereinheiten 27.1 und 27.2 sind mit einem Mikroprozessor 33 gekoppelt, der zum Zweck der Bedienung eine Datenanzeige sowie eine Datenänderung zu jeder der Feldsteuereinheiten 27 ermöglicht. Hierzu ist der Mikroprozessor 33 mit einer bedienbaren Visualisiereinheit 34 verbunden. In den Fällen, in denen eine getrennte Bedienung zur Steuerung und Überwachung der Sektionen gewünscht wird, könnte der Mikroprozessor 33 auch mit zwei separaten Visualisiereinheiten verbunden sein.
Bei der in Fig. 1 und 2 gezeigten Texturiermaschine werden alle Bearbeitungsstellen der Sektion 28.1 unabhängig von den Bearbeitungsstellen benachbarter Sektionen 28.2 usw. durch die Feldsteuereinheit 27.1 überwacht und gesteuert. Jede Sektion bildet eine Teilmaschine, die unabhängig von der benachbarten Sektion individuell betrieben werden kann. Lediglich die Energieversorgung (hier nicht dargestellt) erfolgt für die Sektionen aus einer zentralen Schnittstelle heraus, da die Texturiermaschine an einer Einspeisung der Versorgungsspannung angeschlossen ist. Ebenso werden Einrichtungen, wie beispielsweise die Druckluftversorgung zur Absaugung der Fadenabfälle, aus einer zentralen Schnittstelle heraus versorgt.

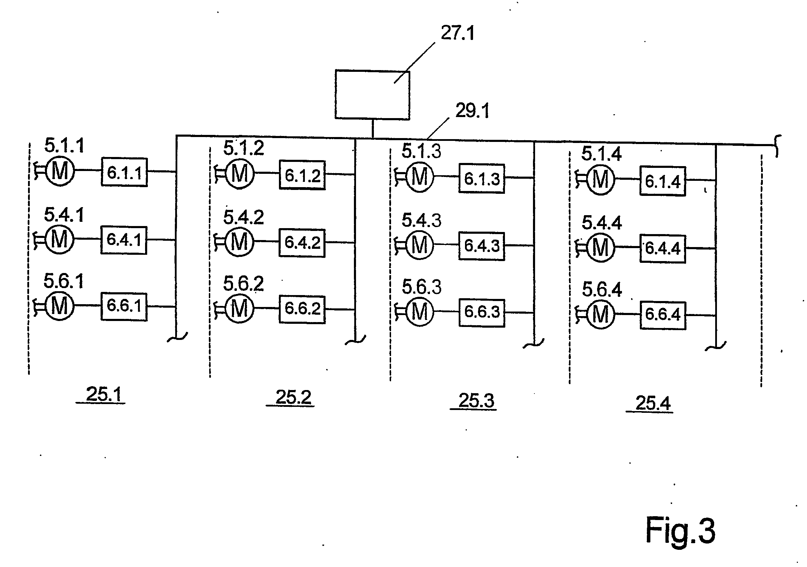

In Fig. 3 ist schematisch die Ankopplung zwischen den Prozeßsteuergeräten der Bearbeitungsstellen einer Sektion mit der Feldsteuereinheit dargestellt, wie sie in der Texturiermaschine aus Fig. 1 und 2 ausgeführt ist. Hierbei sind pro Bearbeitungsstelle 25 nur ein Teil der Bearbeitungsstellen der Sektion und nur ein Teil der Prozeßaggregate einer Bearbeitungsstelle dargestellt. Als Beispiel sind vier Bearbeitungsstellen 25.1, 25.2, 25.3 und 25.4 gewählt. Jede Bearbeitungsstelle enthält zur Förderung und Verstreckung des Fadens insgesamt drei Prozeßaggregate 5.1, 5.4 und 5.6, die als Lieferwerke 11, 15 und 17 ausgebildet sind. In dem Schema in Fig. 3 sind aufgrund der Übersicht nur die Antriebe der Prozeßaggregate dargestellt. Die Bearbeitungsstelle 25.1 enthält somit die Prozeßaggregate 5.1.1, 5.4.1 und 5.6.1. die Bearbeitungsstelle 25.2 enthält ebenfalls die gleichen Prozeßaggregate 5.1.2, 5.4.2 und 5.6.2. Analog sind die Prozeßaggregate zu den Bearbeitungsstellen 25.1 und 25.4 angeordnet. Jedem Prozeßaggregat 5.1 ist jeweils ein einzelnes Prozeßsteuergerät 6.1 zugeordnet, so daß in der Bearbeitungsstelle 25.1 das Prozeßaggregat 5.1.1 mit dem Prozeßsteuergerät 6.1.1 gekoppelt ist. Entsprechend ist das Prozeßaggregat 5.4.1 mit einem Prozeßsteuergerät 6.4.1 und das Prozeßaggregat 5.6.1 mit dem Prozeßsteuergerät 6.6.1 gekoppelt. Die übrigen Bearbeitungsstellen 25.2, 25.3 und 25.4 sind ebenfalls derart aufgebaut, daß jedes Prozeßaggregat 5 mit einem eigenständigen Prozeßsteuergerät 6 gekoppelt ist. In dem gezeigten Ausführungsbeispiel sind als Prozeßaggregate fünf Antriebe gewählt, so daß die Prozeßsteuergeräte 6 durch Umrichter gebildet werden könnten.

Alle Prozeßsteuergeräte 6.1, 6.4 und 6.6 der Bearbeitungsstellen 25.1, 25.2, 25.3 und 25.4 sind alle über das Bussystem 29.1 mit der Feldsteuereinheit 27.1 gekoppelt. Somit läßt sich jedem der Prozeßaggregate in jeder der Bearbeitungsstellen individuell steuern.

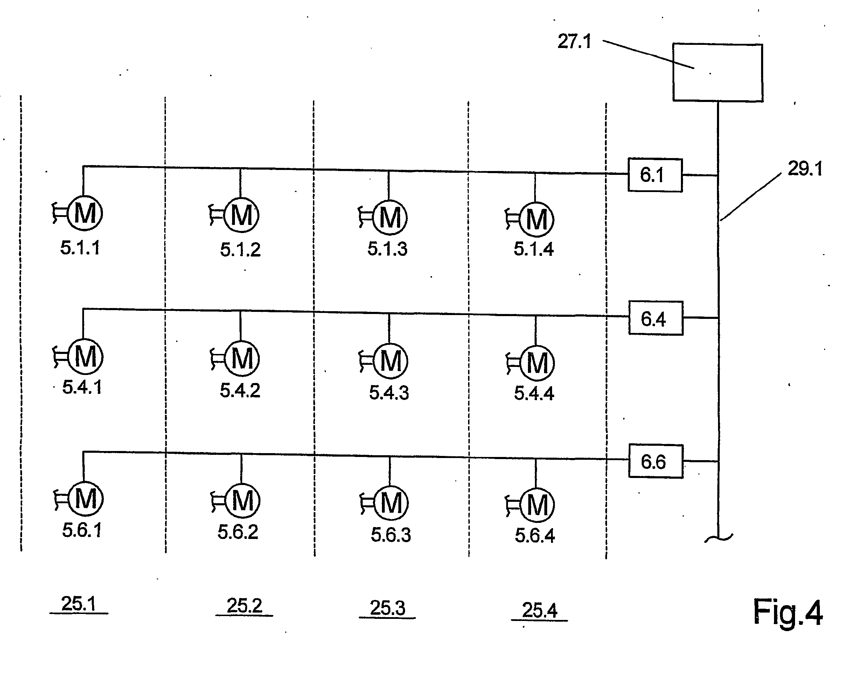

In Fig. 4 ist schematisch ein weiteres Ausführungsbeispiel zur Ansteuerung der Prozeßaggregate innerhalb einer Sektion dargestellt. Die Sektion wird hierbei durch die Feldsteuereinheit 27.1 gesteuert. Als Beispiel sind nur vier Bearbeitungsstellen 25.1, 25.2, 25.3 und 25.4 der Sektion dargestellt. Hierbei wurden wiederum die Antriebe der Lieferwerke 11, 15 und 17 beispielhaft als Prozeßaggregate gewählt. Die Bearbeitungsstelle 25.1 weist somit die Prozeßaggregate 5.1.1, 5.4.1, 5.6.1 und die Bearbeitungsstelle 25.2 die Prozeßaggregate 5.1.2., 5.4.2 und 5.6.2 auf. Die weiteren Bearbeitungsstellen sind entsprechend aufgebaut. Die Prozeßaggregate 5.1.1, 5.1.2, 5.1.3 und 5.1.4 der Bearbeitungsstellen 25.1, 25.2, 25.3 und 25.4 sind zu einer Gruppe zusammengefaßt und werden gemeinsam über das Prozeßsteuergerät 6.1 angesteuert. Ebenso sind die Prozeßaggregate 5.4 der Bearbeitungsstellen 25.1, 25.2, 25.3 und 25.4 zu einer Gruppe zusammengefaßt und mit dem Prozeßsteuergerät 6.4 gekoppelt. Die Prozeßaggregate 5.6 aller Bearbeitungsstellen sind mit dem Prozeßsteuergerät 6.6 verbunden. Über das Bussystem 29.1 sind die Prozeßsteuergeräte 6.1, 6.4 und 6.6 mit der Feldsteuereinheit 27.1 gekoppelt. Eine derartige Ankopplung der Prozeßaggregate ist insbesondere für Antriebe der Bearbeitungsstellen vorteilhaft, die mit gleicher Geschwindigkeit betrieben werden. Die Prozeßsteuergeräte 6.1, 6.4 oder 6.6 stellen somit einen Gruppenumrichter dar, der alle angeschlossenen Antriebe gleichermaßen steuert.

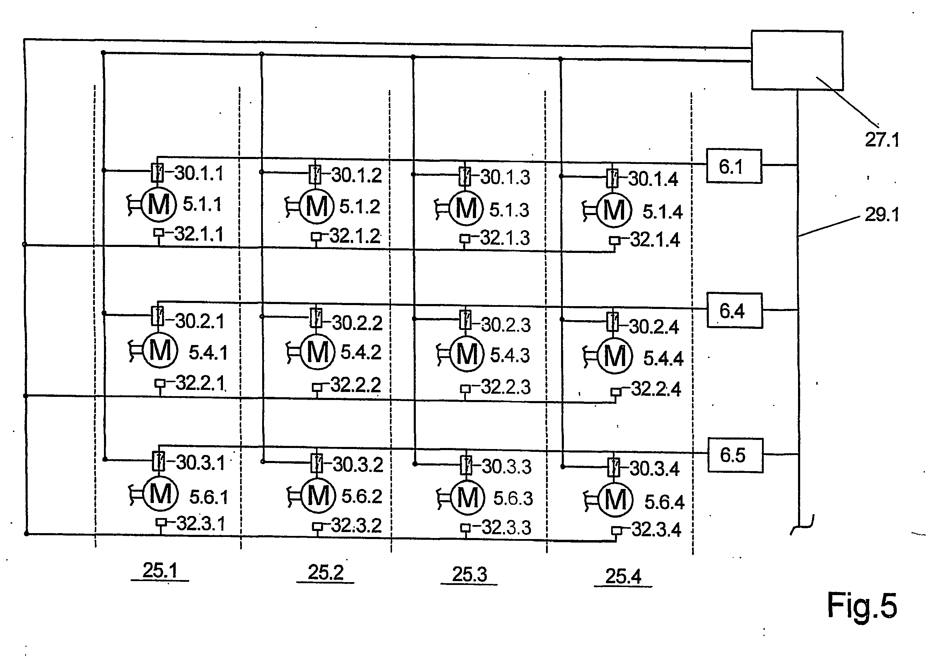

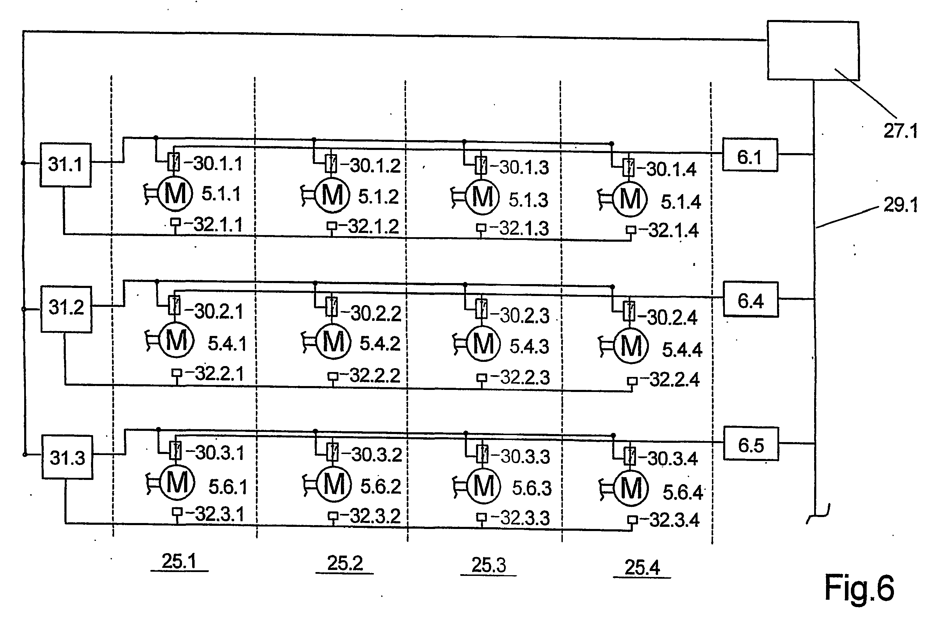

In Fig. 5 und 6 sind weitere Beispiele der Ankopplung und Überwachung der Prozeßaggregate einer Sektion an einer Feldsteuereinheit schematisch dargestellt. Die in Fig. 5 gezeigte Ausführung ist mit der in Fig. 4 gezeigten Ankopplung identisch. Insoweit wird auf die vorhergehende Beschreibung Bezug genommen, und nur die Unterschiede werden nachfolgend angegeben.

Die Prozeßaggregate 5.1 der Bearbeitungsstellen 25.1 bis 25.4 sind gemeinsam an dem Prozeßsteuergerät 6.1 angeschlossen. Dabei ist jedem Prozeßaggregat 5.1 ein Schalter 30.1 zugeordnet. Durch den Schalter 30.1 läßt sich die Verbindung zwischen dem Prozeßsteuergerät 6.1 und dem jeweiligen Prozeßaggregat 5.1 unterbrechen. Hierzu wird der Schalter 30.1 unmittelbar von der Feldsteuereinheit 27.1 angesteuert. Jedem der Prozeßaggregate 5.1 ist eine Sensorik 32.1 zugeordnet, die jeweils mit der Feldsteuereinheit 27.1 verbunden ist. In der gleichen Weise sind den Prozeßaggregaten 5.4 in den Bearbeitungsstellen 25.1, 25.2, 25.3 und 25.4 jeweils ein Schalter 30.2 und eine Sensorik 32.2 zugeordnet. Ebenso sind die Prozeßaggregate 5.6 der vier Bearbeitungsstellen 25.1, 25.2, 25.3 und 25.4 über jeweils einen Schalter 30.3 mit dem Prozeßsteuergerät 6.6 gekoppelt. Den Prozeßaggregaten 5.6 sind des weiteren die Sensoren 32.3.1 bis 32.3.4 zugewiesen.

Bei der in Fig. 5 gezeigten Ausführung der erfindungsgemäßen Texturiermaschine wird über die Sensorik 32 zumindest ein Parameter in dem Prozeßaggregat 5 überwacht. Dies kann beispielsweise eine Drehzahlüberwachung oder eine Temperaturüberwachung sein. Die aktuell erfaßten Daten werden durch die Sensorik 32 der Feldsteuereinheit 27.1 aufgegeben. Für den Fall, daß eine der Prozeßaggregate 5 einen unzulässigen Parameterwert aufweist, erfolgt eine Aktivierung des dem Prozeßaggregat 5 zugeordneten Schalters 30.1 durch die Feldsteuereinheit 27.1. Das betreffende Prozeßaggregat 5 wird somit unverzüglich von dem Prozeßsteuergerät 6 abgekoppelt. Dadurch können beispielsweise Fadenbrüche in einer Bearbeitungsstelle ohne Beeinflussung der benachbarten Beeinflussungsstellen, die dem gleichen Steuergerät zugeordnet sind, behoben werden.

In Fig. 6 ist ein weiteres Ausführungsbeispiel einer Steuereinrichtung mit Gruppensteuerung dargestellt. Der Aufbau und die Anordnung der Aggregate entsprechen im wesentlichen dem Aufbau des Schemas aus Fig. 5, so daß auf die vorhergehende Beschreibung Bezug genommen wird. Gegenüber dem in Fig. 5 gezeigten Ausführungsbeispiel sind die Schalter 30.1.1 bis 30.1.4 der benachbarten Bearbeitungsstellen 25.1 bis 25.4 an einer Überwachungseinheit 31.1 angeschlossen. Ebenso ist die Sensorik 32.1.1 bis 32.1.4 der benachbarten Bearbeitungsstellen 25.1 bis 25.4 an der Überwachungseinheit 31.1 angebunden. In der Überwachungseinheit 31.1 sind Sollparameter der einzelnen Prozeßaggregate 5.1.1 bis 5.1.4 hinterlegt. Sobald einer der Sensoren 32.1 einen abweichenden Parameter signalisiert, wird über die Überwachungseinheit 31.1 der dem Prozeßaggregat 5.1 zugeordnete Schalter 30.1 aktiviert. In gleicher Weise ist die Steuerung und Überwachung der Prozeßaggregate 5.4 und 5.6 ausgeführt, In den Prozeßaggregaten 5.4 ist die Überwachungseinheit 31.2 und in den Prozeßaggregaten 5.6 die Überwachungseinheit 31.3 zugeordnet. Zum Datenaustausch sowie zum Empfang übergeordneter Steuerungsbefehle sind die Überwachungseinheiten 31.1, 31.2 und 31.3 mit der Feldsteuereinheit 27.1 verbunden.

Bei den zuvor beschriebenen Ausführungsbeispielen der erfindungsgemäßen Texturiermaschine ist die Anzahl der Bearbeitungsstellen innerhalb einer Sektion beispielhaft. So lassen sich ohne Probleme sechs, acht, zehn oder auch mehr Bearbeitungsstellen zu einer Sektion zusammenfassen. Die Sektionen können auch vorteilhaft dadurch gebildet werden, daß vorgegebene Trennungen im Gattergestell als Vorgabe zur Wahl der Anzahl der Bearbeitungsstellen gewählt wird. So ist bekannt, daß Gattergestelle durch mehrere bewegliche Einheiten gebildet werden, wobei jede der Einheiten beispielsweise 12 Vorlagespulen enthält. In diesem Fall entspricht eine Sektion einer Gatterteilung.

Ebenso sind die Anzahl und die Ausführung der Prozeßaggregate beispielhaft. Die Texturiermaschine kann beispielsweise nur zwei Lieferwerke aufweisen, wobei dem zweiten Lieferwerk unmittelbar die Aufwickeleinrichtung nachgeschaltet ist. Es ist jedoch auch möglich, mehrere Lieferwerke als Prozeßaggregate vorzusehen, so daß beispielsweise zwischen dem Falschdrallaggregat und dem Set-Heizer zwei Lieferwerke aufeinander folgen, zwischen denen ein weiteres Prozeßaggregat zur Verwirbelung der Fäden angeordnet ist. Das erfindungsgemäße Verfahren und die erfindungsgemäße Texturiermaschine zeichnen sich unabhängig von der Ausführung der Prozeßaggregate und Prozeßsteuergeräte dadurch aus, daß eine Vielzahl von Bearbeitungsstellen in wenige Sektionen aufgeteilt und als kleine Einheiten auf einfache Art steuerbar sind.

### Bezugszeichenliste

- 1: Wickelgestell
- 2: Gattergestell
- 3: Prozeßgestell
- 4: Faden
- 5: Prozeßaggregat
- 6: Prozeßsteuergerät
- 7: Vorlagespule
- 9: Umlenkrolle
- 10: Kopffadenführer
- 11: Erstes Lieferwerk
- 12: Heizeinrichtung
- 13: Kühlschiene
- 14: Falschdrallaggregat
- 15: Zweites Lieferwerk
- 16: Set-Heizer
- 17: Drittes Lieferwerk
- 18: Spulenantrieb
- 19: Changiervorrichtung
- 20: Aufwickeleinrichtung
- 21: Spulenspeicher
- 22: Hülsenzufuhreinrichtung
- 23: Bediengang
- 24: Doffgang
- 25: Bearbeitungsstelle
- 26: Spule
- 27: Feldsteuereinheit
- 28: Sektion
- 29: Bus-System
- 30: Schalter
- 31: Überwachungseinheit
- 32: Sensorik
- 33: Mikroprozessor
- 34: Visualisiereinheit

## Patentansprüche

1. Verfahren zur Steuerung einer Texturiermaschine zum Falschdralltexturieren einer Vielzahl von synthetischen Fäden mit einer entsprechenden Vielzahl von Bearbeitungsstellen,
**dadurch gekennzeichnet, daß**
die Vielzahl der Bearbeitungsstellen in mehrere Sektionen mit jeweils mehreren Bearbeitungsstellen aufgeteilt ist und daß die Bearbeitungsstellen einer der Sektionen unabhängig von den Bearbeitungsstellen der benachbarten Sektionen überwacht und gesteuert werden.

2. Verfahren nach Anspruch 1,
**dadurch gekennzeichnet, daß**
in jeder Bearbeitungsstelle einer Sektion jeweils ein Faden durch mehrere Prozeßschritte zumindest gefördert, texturiert, verstreckt und aufgewickelt wird und daß alle Prozeßschritte der Sektion durch eine der Sektion zugeteilten Feldsteuereinheit überwacht und beeinflußt werden.

3. Verfahren nach Anspruch 2,
**dadurch gekennzeichnet, daß**
jeder Prozeßschritt einer der Bearbeitungsstellen unabhängig von den benachbarten Prozeßschritten der Bearbeitungsstelle einzeln gesteuert wird.

4. Verfahren nach Anspruch 2 oder 3,
**dadurch gekennzeichnet, daß**
die Prozeßschritte einer der Bearbeitungsstellen unabhängig von den Prozeßschritten der benachbarten Bearbeitungsstellen der Sektion einzeln gesteuert und überwacht werden.

5. Verfahren nach Anspruch 2 oder 3,
**dadurch gekennzeichnet, daß**
die Prozeßschritte benachbarter Bearbeitungsstellen unabhängig von den jeweils benachbarten Prozeßschritten der Bearbeitungsstellen der Sektion als eine Gruppe gesteuert werden.

6. Verfahren nach einem der Ansprüche 2 bis 5,
**dadurch gekennzeichnet, daß**
die Prozeßschritte der Bearbeitungsstellen der Sektion teilweise einzeln und teilweise als Gruppe gesteuert werden.

7. Verfahren nach Anspruch 5 oder 6,
**dadurch gekennzeichnet, daß**
die Prozeßschritte einer Gruppe unabhängig von der Steuerung separat abgeschaltet werden.

8. Verfahren nach Anspruch 7,
**dadurch gekennzeichnet, daß**
das Abschalten eines der Prozeßschritte in Abhängigkeit eines fortlaufend überwachten Parameters erfolgt.

9. Verfahren nach einem der vorgenannten Ansprüche,
**dadurch gekennzeichnet, daß**
zumindest sechs Fäden vorzugsweise zwölf Fäden parallel nebeneinander in den Bearbeitungsstellen einer der Sektionen gleichzeitig texturiert werden.

10. Texturiermaschine zum Falschdralltexturieren einer Vielzahl von synthetischen Fäden (4) mit einer entsprechenden Vielzahl von Bearbeitungsstellen (25), wobei die Fäden (4) in den Bearbeitungsstellen (25) durch mehrere Prozeßaggregate (5) zumindest gefördert, texturiert, verstreckt und aufgewickelt werden, und mit mehreren Prozeßsteuergeräten (6), die den Prozeßaggregaten (5) der Bearbeitungsstellen (25) zur Steuerung zugeordnet sind,
**dadurch gekennzeichnet, daß**
die Vielzahl der Bearbeitungsstellen (25) in mehrere Sektionen (28) mit jeweils mehreren Bearbeitungsstellen (25) aufgeteilt ist, daß jeder Sektion (28) eine von mehreren Feldsteuereinheiten (27) zur Steuerung und Überwachung der Barbeitungsstellen (25) der Sektion (28) zugeordnet ist und daß die Prozeßsteuergeräte (6) der Bearbeitungsstellen (25) einer Sektion (28) mit der der Sektion (28) von Bearbeitungsstellen (25) zugordneten Feldsteuereinheit (27) verbunden sind.

11. Texturiermaschine nach Anspruch 10,
**dadurch gekennzeichnet, daß**
die Feldsteuereinheiten (27) unabhängig voneinander zur Datenanzeige, Dateneingabe und/oder Datenänderung mit einem Mikroprozessor (33) verbunden sind.

12. Texturiermaschine nach Anspruch 11,
**dadurch gekennzeichnet, daß**
der Mikroprozessor (33) über ein Ferndatennetz mit einer Diagnosestation koppelbar ist.

13. Texturiermaschine nach einem der Ansprüche 10 bis 12,
**dadurch gekennzeichnet, daß**
die Feldsteuereinheiten (27) unabhängig voneinander über ein serielles BUS-System (29) mit den zugeordneten Prozeßsteuergeräten (6) verbunden sind.

14. Texturiermaschine nach Anspruch 13,
**dadurch gekennzeichnet, daß**
das BUS-Sytem (29) aus mehreren Datennetzwerken mit unterschiedlichen Datenübertragungsraten gebildet ist.

15. Texturiermaschine nach einem der Ansprüche 10 bis 14,
**dadurch gekennzeichnet, daß**
jedem der Prozeßaggregate (5) der Bearbeitungsstellen (25) einer Sektion (28) ein einzelnes der Prozeßsteuergeräten (6) zugeordnet ist.

16. Texturiermaschine nach einem der Ansprüche 10 bis 14,
**dadurch gekennzeichnet, daß**
die Prozeßaggregate (5) der Bearbeitungsstellen (25) einer Sektion (28) in Gruppen gleicher Funktion aufgeteilt sind und daß jeder Gruppe von Prozeßaggregaten (5) ein einzelnes der Prozeßsteuergeräten (6) zugeordnet ist.

17. Texturiermaschine nach einem der Ansprüche 10 bis 14,
**dadurch gekennzeichnet, daß**
die Prozeßaggregate (5) der Bearbeitungsstellen (25) einer Sektion (28) teilweise einzeln einer der Prozeßsteuergeräten (6) und teilweise in Gruppen einer der Prozeßsteuergeräten (6) zugeordnet sind.

18. Texturiermaschine nach einem der Ansprüche 10 bis 17,
**dadurch gekennzeichnet, daß**
zumindest einem Teil der Prozeßaggregate (5) ein Schalter (30) zugeordnet ist, durch welchen die Prozeßaggregate (5) schaltbar mit einer der Prozeßsteuergeräten (6) verbunden sind, und daß der Schalter (30) unabhängig von dem Prozeßsteuergerät (6) ansteuerbar ist.

19. Texturiermaschine zum Falschdralltexturieren einer Vielzahl von synthetischen Fäden (4) mit einer entsprechenden Vielzahl von Bearbeitungsstellen (25) , wobei die Fäden (4) in den Bearbeitungsstellen (25) durch mehrere Prozeßaggregate (5) zumindest gefördert, texturiert, verstreckt und aufgewickelt werden, und mit mehreren Prozeßsteuergeräten (6), die zur Steuerung den Prozeßaggregaten (5) der Bearbeitungsstellen (25) zugeordnet sind,
**dadurch gekennzeichnet, daß**
in einer Gruppe von Prozeßaggregaten (5) benachbarter Bearbeitungsstellen (25) den Prozeßaggregaten (5) jeweils ein Schalter (30) zugeordnet ist, durch welche die Prozeßaggregate (5) schaltbar mit einer der Prozeßsteuergeräten (6) verbunden sind, und daß die Schalter (30) unabhängig von dem zugeordneten Prozeßsteuergerät (6) ansteuerbar sind.

20. Texturiermaschine nach Anspruch 18 oder 19,
**dadurch gekennzeichnet, daß**
jeder Schalter (30) durch eine dem Prozeßsteuergerät (6) übergeordneten Feldsteuereinheit (27) ansteuerbar ist.

21. Texturiermaschine nach Anspruch 18 oder 19,
**dadurch gekennzeichnet, daß**
jeder Schalter (30) durch eine dem Prozeßaggregat (6) zugeordnete Überwachungseinheit (31) ansteuerbar ist, welche mit einer dem Prozeßsteuergerät (6) übergeordneten Feldsteuereinheit (27) verbunden ist.

22. Texturiermaschine nach Anspruch 20 oder 21,
**dadurch gekennzeichnet, daß**
eine Sensorik (32) zur Überwachung zumindest eines Parameters des Prozeßaggregates (5) vorgesehen ist und daß die Sensorik (32) mit der dem Prozeßaggregat (5) zugeordneten Überwachungseinheit (31) oder mit der dem Prozeßaggregat (5) zugeordneten Feldsteuereinheit (27) verbunden ist.

23. Texturiermaschine nach einem der Ansprüche 10 bis 22,
**dadurch gekennzeichnet, daß**
die Sektionen (28) jeweils zumindest sechs Bearbeitungsstellen (25) vorzugsweise zwölf Bearbeitungsstellen (25) enthalten.

24. Texturiermaschine nach einem der Ansprüche 11 bis 23,
**dadurch gekennzeichnet, daß**
der Mikroprozessor (33) zur Steuerung und Überwachung der Sektionen (28) mit einer bedienbaren Visualisiereinheit (34) oder maximal mit zwei bedienbaren Visualisiereinheiten (34) verbunden ist.

## Claims

1. Method of controlling a texturing machine for false twist texturing a plurality of synthetic filaments yarns with a corresponding plurality of processing stations,
**characterized in that**
the plurality of processing stations are divided into a plurality of sections, each section comprising a plurality of processing stations, and that the processing stations of one of the sections are monitored and controlled independently of the processing stations of adjacent sections.

2. Method of claim 1,
**characterized in that**
in each processing station of a section, one yarn each is at least advanced, textured, drawn, and wound by a plurality of process steps, and that all process steps of the section are monitored and influenced by a field control unit associated to the section.

3. Method of claim 2,
**characterized in that**
each process step of one of the processing stations is individually controlled irrespective of the closest process steps of the processing station.

4. Method of claim 2 or 3,
**characterized in that**
the process steps of one of the processing stations are individually controlled and monitored irrespective of the process steps of adjacent processing stations of the section.

5. Method of claim 2 or 3,
**characterized in that**
the process steps of adjacent processing stations are controlled as a group irrespective of respectively closest processing steps of the processing stations of the section.

6. Method of one of claims 2-5,
**characterized in that**
the process steps of the processing stations of the section are controlled in part individually and in part as a group.

7. Method of claim 5 or 6,
**characterized in that**
the process steps of a group are separately shut down irrespective of the control.

8. Method of claim 7,
**characterized in that**
the shutdown of one of the process steps occurs as a function of a continuously monitored parameter.

9. Method of one of the foregoing claims,
**characterized in that**
in the processing stations of one of the sections, at least six yarns, preferably twelve yarns, are simultaneously textured in parallel, side-by-side relationship.

10. Texturing machine for false twist texturing a plurality of synthetic filament yarns (4) with a corresponding plurality of processing stations (25), the yarns (4) in the processing stations (25) being at least advanced, textured, drawn, and wound by a plurality of processing units (5), and with a plurality of process controllers (6), which are associated to the processing units (5) of the processing stations (25),
**characterized in that**
the plurality of processing stations (25) are divided into a plurality of sections (28), with each section comprising a plurality of processing stations (25), that to each section (28) one of a plurality of field control units (27) is associated for controlling and monitoring the processing stations (25) of the section (28), and that the process controllers (6) of the processing stations (25) of a section (28) connect to the field control unit (27) associated to the section (28) of the processing stations (25).

11. Texturing machine of claim 10,
**characterized in that**
the field control units (27) are connected independently of one another to a microprocessor (33) for displaying, inputting, and/or changing data.

12. Texturing machine of claim 11,
**characterized in that**
the microprocessor (33) can be coupled via a line data network to a diagnosis station.

13. Texturing machine of one of claims 10-12,
**characterized in that**
the field control units (27) connect independently of one another, via a serial bus system (29), to the associated process controllers (6).

14. Texturing machine of claim 13,
**characterized in that**
the bus system (29) is formed from a plurality of data networks with different data transmission rates.

15. Texturing machine of one of claims 10-14,
**characterized in that**
an individual one of the process controllers (6) is associated to each of the processing units (5) of the processing stations (25) of a section (28).

16. Texturing machine of one of claims 10-14,
**characterized in that**
the processing units (5) of the processing stations (25) of a section (28) are divided into groups of the same function, and that an individual one of the process controllers (6) is associated to each group of processing units (5)

17. Texturing machine of one of claims 10-14,
**characterized in that**
the processing units (5) of the processing stations (25) of a section (28) are associated in part individually to one of the process controllers (6) and in part in groups to one of the process controllers (6).

18. Texturing machine of one of claims 10-17,
**characterized in that**
to at least one part of the processing units (5) a switch (30) is associated, by which the processing units (5) are switchably connected to one of the process controllers (6), and that the switch (30) is activatable independently of the process controller (6).

19. Texturing machine for false twist texturing a plurality of synthetic filaments yarns(4) with a corresponding plurality of processing stations (25), wherein the yarns (4) in the processing stations (25) are at least advanced, textured, drawn, and wound by a plurality of processing units (5), and with a plurality of process controllers (6), which are associated for controlling to the processing units (5) of the processing stations (25),
**characterized in that**
in a group of processing units (5) of adjacent processing stations (25) respectively one switch (30) is associated to the processing units (5), the switch connecting the processing units (5) in a switchable manner to one of the process controllers (6), and that the switches (30) are activatable independently of the associated process controller (6).

20. Texturing machine of claim 18 or 19,
**characterized in that**
each switch (30) is activatable by a field control unit (27) overriding the process controller (6).

21. Texturing machine of claim 18 or 19,
**characterized in that**
each switch (30) is activatable by a monitoring unit (31) associated to the process controller (6), the monitoring unit connecting to a field control unit (27) overriding the process controller (6).

22. Texturing machine of claim 20 or 21,
**characterized in that**
a sensor arrangement (32) is provided for monitoring at least one parameter of the processing unit (5), and that the sensor arrangement (32) connects to the monitoring unit (31) associated to the processing unit (5), or to the field control unit (27) associated to the processing unit (5).

23. Texturing machine of one of claims 10-22,
**characterized in that**
the sections (28) contain each at least six processing stations (25), preferably twelve processing stations (25).

24. Texturing machine of one of claims 11-23,
**characterized in that**
the microprocessor (33) for controlling and monitoring sections (28) connects to an operator-controllable visual display unit (34) or at most to two operator-controllable visual display units (34).

## Revendications

1. Procédé destiné à la commande d'une machine à texturer pour la texturation par fausse torsion d'une multiplicité de fils synthétiques avec une multiplicité correspondante de postes de traitement, **caractérisé en ce que** la multiplicité des postes de traitement est répartie en plusieurs sections avec respectivement plusieurs postes de traitement et **en ce que** les postes de traitement d'une des sections sont contrôlés et commandés indépendamment des postes de traitement des sections adjacentes.

2. Procédé selon la revendication 1, **caractérisé en ce que** dans chaque poste de traitement d'une section un fil respectif est au moins avancé, texturé, étiré et enroulé par plusieurs étapes de processus et **en ce que** toutes les étapes de processus de la section sont contrôlées et influencées par une unité de commande de champ attribuée à la section.

3. Procédé selon la revendication 2, **caractérisé en ce que** chaque étape de processus d'un des postes de traitement est commandée individuellement, indépendamment des étapes de processus adjacentes du poste de traitement.

4. Procédé selon la revendication 2 ou 3, **caractérisé en ce que** les étapes de processus d'un des postes de traitement sont commandées et contrôlées individuellement, indépendamment des étapes de processus des postes de traitement adjacents de la section.

5. Procédé selon la revendication 2 ou 3, **caractérisé en ce que** les étapes de processus de postes de traitement adjacents sont commandées comme un groupe, indépendamment des étapes de processus respectivement adjacentes des postes de traitement de la section.

6. Procédé selon l'une des revendications 2 à 5, **caractérisé en ce que** les étapes de processus des postes de traitement de la section sont commandées partiellement de manière individuelle et partiellement comme groupe.

7. Procédé selon la revendication 5 ou 6, **caractérisé en ce que** les étapes de processus d'un groupe sont débranchées séparément indépendamment de la commande.

8. Procédé selon la revendication 7, **caractérisé en ce que** le débranchement d'une des étapes de processus est effectué en fonction d'un paramètre surveillé continuellement.

9. Procédé selon l'une des revendications précédentes, **caractérisé en ce qu'**au moins six fils, de préférence 12 fils, sont texturés en même temps parallèlement les uns à coté des autres dans les postes de traitement d'une des sections.

10. Machine à texturer pour texturer par fausse torsion une multiplicité de fils synthétiques (4) avec une multiplicité correspondante de postes de traitement (25), les fils (4) étant au moins avancés, texturés, étirés et enroulés par plusieurs agrégats de processus (5) et avec plusieurs dispositifs de commande de processus (6) qui sont associés aux agrégats de processus (5) des postes de traitement (25) pour être commandés, **caractérisée en ce que** la multiplicité des postes de traitement (25) est subdivisée en plusieurs sections (28) avec respectivement plusieurs postes de traitement (25), **en ce qu'**à chaque section (28) on a associé une de plusieurs unités de commande de champ (27) pour commander et pour surveiller les postes de traitement (25) de la section (28) et **en ce que** les dispositifs de commande de processus (6) des postes de traitement (25) d'une section (28) sont connectés à l'unité de commande de champ (27) associée à la section (28) de postes de traitement (25).

11. Machine à texturer selon la revendication 10, **caractérisée en ce que** les unités de commande de champ (27) sont connectées indépendamment l'une de l'autre pour l'indication de données, l'entrée des données et/ou la modification de données à un microprocesseur (33).

12. Machine à texturer selon la revendication 11, **caractérisée en ce que** le microprocesseur (33) peut être accouplé à une station de diagnostic à l'intermédiaire d'un réseau de données télétransmises.

13. Machine à texturer selon l'une des revendications 10 à 12, **caractérisée en ce que** les unités de commande de champ (27) sont connectées aux dispositifs de commande de processus (6) à l'intermédiaire d'un système BUS en série (29) indépendamment l'une de l'autre.

14. Machine à texturer selon la revendication 13, **caractérisée en ce que** le système BUS (29) est formé de plusieurs réseaux de données avec des débits numériques de données différents.

15. Machine à texturer selon l'une des revendications 10 à 14, **caractérisée en ce qu'**un dispositif de commande de processus (6) individuel est associé à chaque agrégat de processus (5) des postes de traitement (25) d'une section (28).

16. Machine à texturer selon l'une des revendications 10 à 14, **caractérisée en ce que** les agrégats de processus (5) des postes de traitement (25) d'une section (28) sont subdivisés en groupes de fonction identique et **en ce qu'**à chaque groupe d'agrégats de processus (5) on a associé un dispositif de commande de processus (6) individuel.

17. Machine à texturer selon l'une des revendications 10 à 14, **caractérisée en ce que** les agrégats de processus (5) des postes de traitement (25) d'une section (28) sont associés partiellement de façon individuelle à un des dispositifs de commande de processus (6) et partiellement en groupes à un des dispositifs de commande de processus (6).

18. Machine à texturer selon l'une des revendications 10 à 17, **caractérisée en ce qu'**un interrupteur (30) est associé au moins à une partie des agrégats de processus (5), par lequel interrupteur (30) les agrégats de processus (5) sont connectés de manière connectable à un des dispositifs de commande de processus (6) et **en ce que** l'interrupteur (30) peut être activé indépendamment du dispositif de commande de processus (6).

19. Machine à texturer pour la texturation par fausse torsion d'une multiplicité de fils synthétiques (4) avec une multiplicité correspondante de postes de traitement (25), les fils (4) étant au moins avancés, texturés, étirés et enroulés dans les postes de traitement (25) par plusieurs agrégats de processus (5) et avec plusieurs dispositifs de commande de processus (6) qui pour être commandés sont associés aux agrégats de processus (5) des postes de traitement (25), **caractérisée en ce que** dans un groupe d'agrégats de processus (5) de postes de traitement adjacents (25) respectivement un interrupteur (30) est associé aux agrégats de processus (5), interrupteurs par lesquels les agrégats de processus (5) sont reliés de façon connectable à un des dispositifs de commande de processus (6) et **en ce que** les interrupteurs (30) peuvent être activés indépendamment du dispositif de commande de processus (6).

20. Machine à texturer selon la revendication 18 ou 19, **caractérisée en ce que** chaque interrupteur (30) peut être excité par une unité de commande de champ (27) qui est supérieure au dispositif de commande de processus (6).

21. Machine à texturer selon la revendication 18 ou 19, **caractérisée en ce que** chaque interrupteur (30) peut être excité par une unité de contrôle (31) qui est associée à l'agrégat de processus (6), laquelle unité de contrôle est reliée à une unité de commande de champ (27) qui est supérieure au dispositif de commande de processus (6).

22. Machine à texturer selon la revendication 20 ou 21, **caractérisée en ce qu'**une technique sensorielle (32) est prévue pour contrôler au moins un paramètre de l'agrégat de processus (5) et **en ce que** la technique sensorielle (32) est reliée à l'unité de contrôle (31) associée à l'agrégat de processus (5) ou à l'unité de commande de champ (27) associée à l'agrégat de processus (5).

23. Machine à texturer selon l'une des revendications 10 à 22, **caractérisée en ce que** les sections (28) contiennent respectivement au moins six postes de traitement (25), de préférence douze postes de traitement (25).

24. Machine à texturer selon l'une des revendications 11 à 23, **caractérisée en ce que** pour commander et pour contrôler les sections (28) le microprocesseur (33) est relié à une unité de visualisation (34) pouvant être opérée ou maximalement à deux unités de visualisation (34) pouvant être opérées.
